# EUROPEAN PATENT SPECIFICATION

(11) **EP 3 047 797 B1**
(45) Date of publication and mention of the grant of the patent: **18.07.2018**
(21) Application number: 13893765.1
(22) Date of filing: 18.09.2013
(51) Int. Cl.: A61B 5/053, A61B 5/20, A61B 5/05, A61B 10/00

(54) **CURRENT PERCEPTION THRESHOLD EXAMINATION APPARATUS USED IN CATHETER FOR DIAGNOSIS OF INTERSTITIAL CYSTITIS**
VORRICHTUNG ZUR UNTERSUCHUNG EINER STROMERFASSUNGSSCHWELLE IN EINEM KATHETER ZUR DIAGNOSE VON INTERSTITIELLER ZYSTITIS
APPAREIL POUR L'EXAMEN DU SEUIL DE PERCEPTION DU COURANT UTILISÉ DANS DES CATHÉTERS POUR LE DIAGNOSTIC DE LA CYSTITE INTERSTITIELLE

(43) Date of publication of application: 27.07.2016
(73) Proprietor: Tsukada Medical Research Co., Ltd., Tokyo 161-0034 (JP)
(72) Inventor: TSUKADA, Osamu, Ueda-shi Nagano 386-0002 (JP); NAKAJIMA, Yasuhiko, Hiratsuka-shi Kanagawa 254-0013 (JP); UEDA, Tomohiro, Kyoto-shi Kyoto 612-8469 (JP); KUSANO, Kazutoshi, Ueda-shi Nagano 386-0024 (JP); SHIMIZU, Masato, Ueda-shi Nagano 386-2202 (JP)
(74) Representative: Barker Brettell LLP
(86) International application number: PCT/JP2013/075179
(87) International publication number: WO 2015/040702

(56) References cited:
- WO-A1-97/06730
- WO-A1-2004/043260
- WO-A1-2008/039111
- WO-A1-2012/009487
- JP-A- 2008 212 465
- JP-A- 2009 000 268
- US-A1- 2008 319 348
- Katims, J.J.: "Electrodiagnostic functional sensory evaluation of the patient with pain: a review of the neuroselective current perception threshold and pain tolerance threshold", Pain Digest, vol. 8 1 January 1998 (1998-01-01), pages 219-230, XP055224296, Springer, New Yourk Retrieved from the Internet: URL:http://www.eng.umd.edu/~neil/siddharth /childrens/Katims_Neuroselective_CPT_-_Pai n_Digest_1998.pdf [retrieved on 2015-10-28]

## Description

### TECHNICAL FIELD

The present invention relates to a current perception threshold examination apparatus, and more particularly relates to a current perception threshold examination apparatus used in diagnosis of interstitial cystitis with a catheter for diagnosis of interstitial cystitis equipped with electrodes.

### BACKGROUND ART

Catheters with electrodes are well known and are widely used for diagnosis or treatment of neurogenic bladder, urinary incontinence and the like. The catheters with electrodes are sometimes used in diagnosis of interstitial cystitis. A conventional catheter with electrodes has exposed hard electrodes, and is not suitable for insertion into a bladder side from a urethra. Further, there is a catheter with an inflatable balloon attached on an outer circumferential face of the catheter, for the purpose of fixing a position of the catheter.

In recent years, there has been a current perception threshold examination apparatus ("CPT" (Current Perception Threshold) examination apparatus) which has been developed for diagnosing abnormality of a peripheral nerve. This examination apparatus evaluates an amount of a current stimulation at the lowest level that an examinee can feel, by sticking a pair of electrodes onto a skin, and causing a weak alternating current to flow thereto. The CPT examination apparatuses are widely used in measurement of a treatment effect (anesthesia), measurement of an affected site (plastic surgery), evaluation of diabetic peripheral neuropathy (medicine), quantitative evaluation of a perceptive nerve (neurology), diagnosis for distinguishing a disease between impotence due to neuropathy and psychogenic impotence (urology), evaluation of injury and perception (dentistry), quantitative measurement of a pharmacological effect (pharmacology), and the like. Such apparatus is described in WO 97/06730.

The above-described CPT examination apparatus is also used in urology, but is used generally in diagnosis from outside a body, and is not used so much in diagnosis of interstitial cystitis. Interstitial cystitis is a disease that has been found recently. Although there are many potential patients, a deterministic diagnostic method has not been found yet.

Interstitial cystitis is often found in women in their 20's through 60's. Interstitial cystitis often appears as a symptom such as a pain in an upper part of a pubic bone, pollakiuria, or urinary urgency. In a typical mucosa observation, an ulcer appears linearly on a bladder mucosa. Even in a lighter symptom, a petechiae appears on a substantially wide area of the bladder mucosa. General inflammation is a phenomenon that occurs between the time when tissue is injured and the time when the injured tissue is repaired. However, tissue repair continues in the interstitial cystitis.

Since pathology of interstitial cystitis has not been resolved yet, a common reference of diagnosis has not been proposed. As common methods for diagnosing interstitial cystitis, there are endoscopy using a bladder mirror, observation of an interior of a bladder using inflation by water pressure, a bladder biopsy in which inflammatory tissue is removed outside and examined, and the like. However, any one of the diagnosis methods cannot be said as simple and precise.

In the light of the above-described circumstances, the applicant filed patent application concerning a balloon catheter with electrodes under the title of "DIAGNOSIS CATHETER FOR INTERSTITIAL CYSTITIS", which was internationally laid open as International Publication No. WO 2004/043260 on May 27, 2004, and was registered as Japanese Patent No. 3921221 on February 23, 2007. The diagnosis catheter for interstitial cystitis is such that a pair of electrodes is provided on an outer circumferential face of the catheter, and a current perception threshold examination apparatus is connected to the respective electrodes via lead wires. In actual diagnosis, a predetermined current is caused to flow across the electrodes, the value of the current which can be perceived by a patient is recorded, and from the result, whether interstitial cystitis or not is determined.

An initial symptom of interstitial cystitis is a hypersensitivity in a urethra bladder. As a conventional simple examination method, a method for injecting KCl into a bladder is widely adopted. However, the method induces a pain, the pain continues after injection of the KCl, and therefore the method cannot be said as minimally invasive examination. As for the KCl, minimally invasive examination is more important for interstitial cystitis that can be moderated by filling C-fiber. Therefore, the applicant developed the diagnosis catheter for interstitial cystitis disclosed in WO 2004/043260 described above, and has performed diagnosis by using an existing current perception threshold examination apparatus.

### SUMMARY OF INVENTION

### TECHNICAL PROBLEM

However, in the conventional current perception threshold examination apparatus, measurement on a body skin surface has been assumed. Consequently, only an alternating current has been outputted, and a proper value for diagnosis has needed to be found by gradually varying the current. This has been needed to be manually performed by a doctor or the like, and diagnosis has taken a long time.

### SOLUTION TO PROBLEM

The present invention is made in the light of the above-described problem, and is a current perception threshold examination apparatus that controls supply of a current from a power supply to electrodes of a catheter for diagnosis of interstitial cystitis, and the current perception threshold examination apparatus adopts a configuration having a direct current generating unit for generating a direct current on the basis of an instruction of a control unit, and a direct current resistance measuring unit that measures an electric resistance value of a diagnosis site on the basis of the direct current which is supplied to the electrodes, and wherein the current perception threshold examination apparatus adopts a configuration further including an alternating current generating unit for generating an alternating current to be supplied for diagnosis, on the basis of the electric resistance value which is measured.

Further, the current perception threshold examination apparatus adopts a configuration wherein the control unit determines an alternating-current voltage value suitable for diagnosis on the basis of the electric resistance value, and the alternating current generating unit applies the determined voltage to the electrodes in response to an instruction of the control unit.

Further, the current perception threshold examination apparatus adopts a configuration wherein the control unit has a frequency and current value regulating function of regulating a frequency and a current value.

Further, the current perception threshold examination apparatus adopts a configuration wherein the diagnosis of the interstitial cystitis is divided into preliminary examination and regular diagnosis, and the control unit further has a resolution switching function of switching a resolution for a current value in the preliminary examination and the regular diagnosis.

Further, the current perception threshold examination apparatus adopts a configuration wherein a resolution in the regular diagnosis is higher than a resolution in the preliminary examination.

Further, in an example not forming part of the claimed invention, a diagnostic method for diagnosing interstitial cystitis by using the above-described current perception threshold examination apparatus, and a catheter are disclosed, for diagnosis of interstitial castitis. The catheter is connected to the current perception threshold examination apparatus and adopts a configuration of supplying a direct current to a diagnosis site to measure an electric resistance value of the diagnosis site, determining an alternating-current voltage value suitable for diagnosis on the basis of the measured electric resistance value, and applying the determined alternating-current voltage to the electrodes, and regulating a current value and a frequency to diagnose interstitial cystitis on the basis of a current value and a frequency at a time of an examinee feeling a stimulus.

Furthermore, the diagnostic method for diagnosing interstitial, not forming part of the claimed invention, adopts a configuration wherein diagnosis of interstitial cystitis by an alternating current is divided into preliminary examination and regular diagnosis, and a resolution of current value regulation in the regular diagnosis is higher than a resolution in the preliminary examination.

### ADVANTAGEOUS EFFECT OF INVENTION

In the invention of the present application, in order to enable measurement inside a body, it is possible to measure the resistance value of the diagnosis site with a weak direct current in the preliminary examination step, and automatically set application current and voltage in the regular diagnosis on the basis of the measured value, and increased efficiency of diagnosis and reduction of burden on an examinee can be realized.

### BRIEF DESCRIPTION OF THE DRAWINGS

Figure 1 is a schematic vertical sectional view of a conventional catheter for diagnosis of interstitial cystitis disclosed as a reference example.
Figure 2 is a cross sectional view of the catheter seen from a line II-II in Figure 1.
Figure 3 is a cross sectional view of the catheter seen from a line III-III in Figure 1.
Figure 4 is a cross sectional view of the catheter seen from a line IV-IV in Figure 1.
Figure 5 is a partial plan view showing various modification examples of electrodes of the catheter disclosed in Figure 1.
Figure 6 is a schematic explanatory view of an example in which the catheter disclosed in Figure 1 is used in diagnosis of interstitial cystitis.

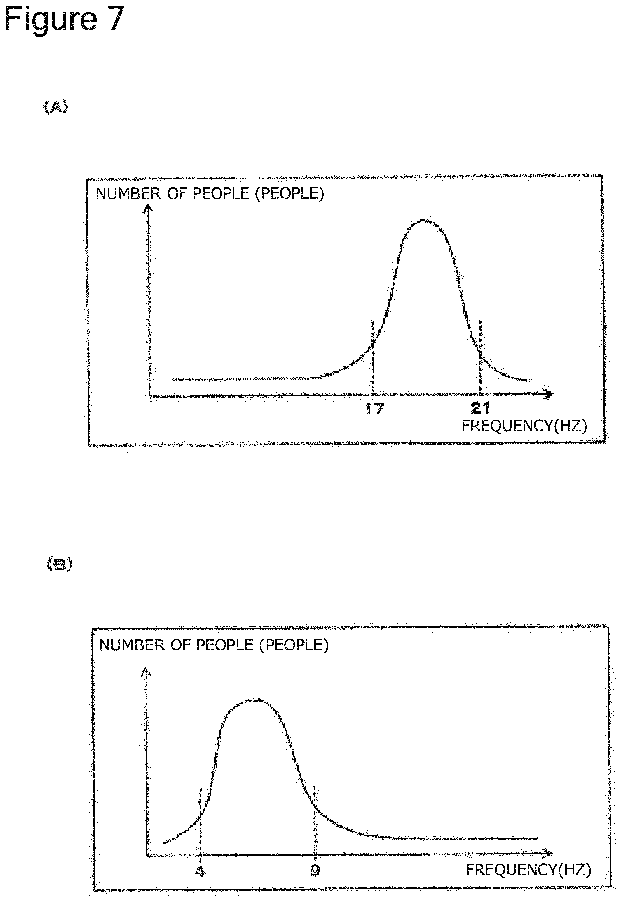

Figure 7 is a graph of a diagnosis result of interstitial cystitis appearing in a display unit of a current perception threshold measurement apparatus, (A) shows a normal value, and (B) shows an abnormal value due to interstitial cystitis, respectively.
Figure 8 is a catheter for diagnosis of interstitial cystitis according to a first embodiment of a reference invention, Figure 8(A) shows a front view, and Figure 8(B) shows a section in a longitudinal direction.
Figure 9(A) is a sectional view showing a state where diagnosis of a bladder is performed with the catheter for diagnosis of interstitial cystitis disclosed in Figure 8, and Figure 9(B) is a sectional view showing a catheter in which electrodes are modified.
Figure 10 is a sectional view of a catheter for diagnosis of interstitial cystitis according to a second embodiment of the reference invention, Figure 10(A) shows a state where a balloon is not inflated, and Figure 10(B) shows a state where the balloon is inflated and a catheter main body is bent.
Figure 11 is a sectional view showing a state where diagnosis of a bladder is performed with the catheter for diagnosis of interstitial cystitis disclosed in Figure 10.
Figure 12 is a sectional view of a catheter for diagnosis of interstitial cystitis according to a modification example of the second embodiment, Figure 12(A) is a sectional view in a longitudinal direction, and Figure 12(B) is a sectional view in a line B to B in Figure 12(A).
Figure 13 is a sectional view of a catheter for diagnosis of interstitial cystitis according to another modification example of the second embodiment, Figure 13(A) is a sectional view in a longitudinal direction, and Figure 13(B) is a sectional view in a line B to B in Figure 13(A).
Figure 14 is a front view showing a catheter for vaginal diagnosis.
Figure 15 is a view showing a current perception threshold measurement apparatus according to one embodiment of the present invention, Figure 15(A) shows a front view, and Figure 15(B) shows a rear view.
Figure 16 is a schematic block diagram explaining an internal circuit configuration of the current perception threshold measurement apparatus disclosed in Figure 15.
Figure 17 is a flowchart showing a diagnosis process of interstitial cystitis by the current perception threshold measurement apparatus disclosed in Figure 15.
Figure 18 is a diagram explaining preliminary examination and regular diagnosis, in diagnosis of interstitial cystitis.

### DESCRIPTION OF EMBODIMENTS

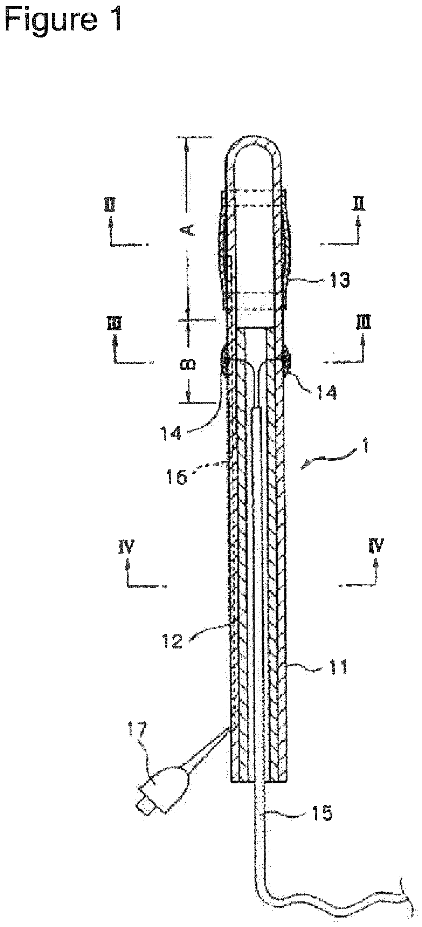

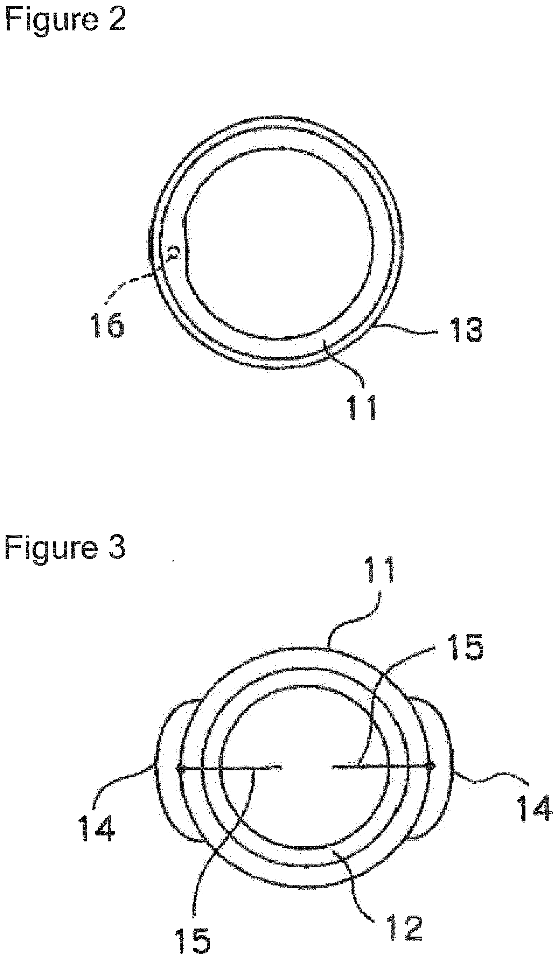

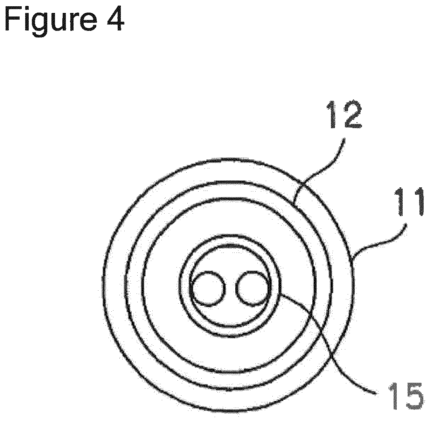

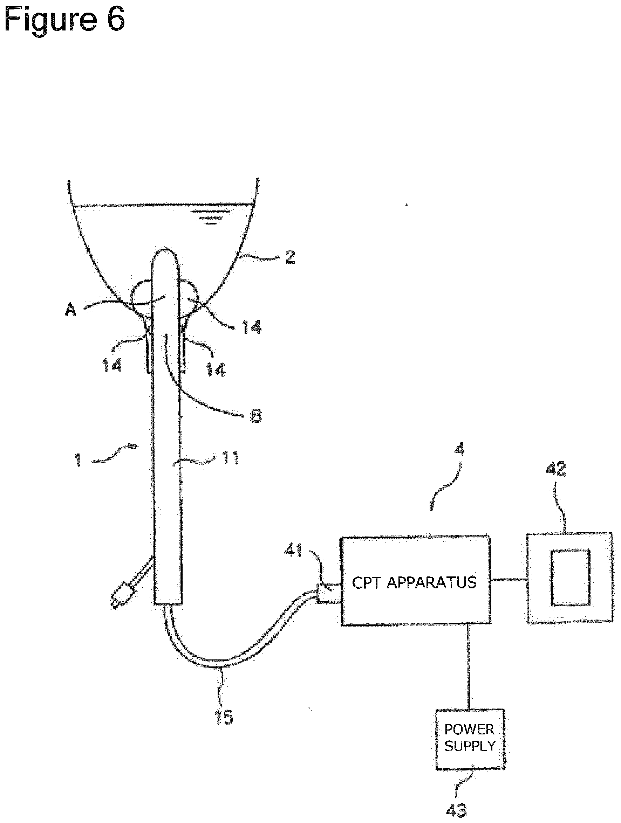

First, referring to Figures 1 to 5, a basic structure of a catheter 1 for diagnosis of interstitial cystitis will be described. As shown in Figure 1, the catheter 1 for diagnosis of interstitial cystitis is connected to a current perception threshold examination apparatus (CPT apparatus) 4 (Figure 6), and is used to diagnose interstitial cystitis.

The catheter 1 for diagnosis of interstitial cystitis mainly includes a catheter main body 11, a core member 12, a balloon 13 capable of inflating and deflating, a pair of electrodes 14, lead wires 15, and a fluid supply passage 16. The catheter main body 11 is made of a soft flexible material (for example, a silicone rubber), and has a bladder-indwelling end portion A and a diagnosis section B adjacent to the distal end section A and at a proximal end side. The core member 12 is made of a rigid flexible member (for example, a polytetrafluoroethylene resin), and is inserted into the diagnosis section in the catheter main body 11. The balloon 13 capable of inflating and deflating is attached to an outer periphery of the bladder-indwelling end portion A of the catheter main body 11.

The pair of electrodes 14 is provided on an outer periphery of the diagnosis section B of the catheter main body 11. The lead wires 15 are inserted into the core member, one ends are connected to the electrodes 14 and the other ends are drawn to outside of a proximal end portion of the catheter main body 11 to be connected to the current perception threshold examination apparatus 4.

The fluid supply passage 16 is provided in the catheter main body 11, a distal end communicates with an interior of the balloon 13, and a proximal end communicates with an injection portion 17. A fluid (for example, air, water, or the like) is supplied to the balloon 13 from the injection portion 17 through the passage 16 to inflate the balloon 13.

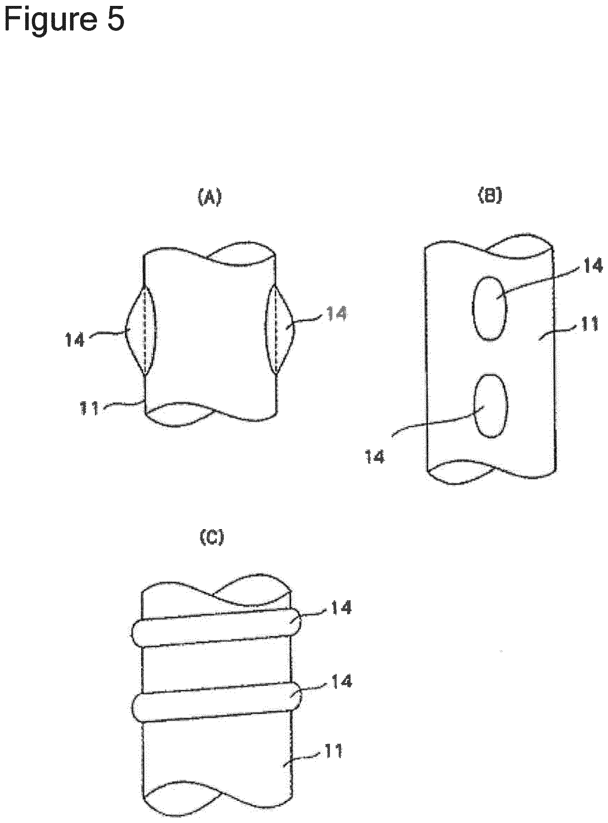

The electrodes 14, as shown in Figure 5, are preferably provided by being separated from each other in a diameter direction of the catheter main body 11 (A), or are provided by being separated from each other in an axial direction of the catheter main body (B), or ring-shaped electrodes that continue in a circumferential direction of the catheter main body 11 are preferably provided by being separated from each other in an axial direction (C).

Next, as shown in Figure 6, the catheter 1 is inserted into a bladder 2 of a patient, the balloon 13 of the catheter 1 is inflated to retain the bladder-indwelling section A of the catheter main body 11 in the bladder 2. At this time, the diagnosis section B of the catheter main body 11 is located in an affected site near an inlet of the bladder 2. The lead wires 15 extending from the proximal end of the catheter 1 are connected to the current perception threshold examination apparatus 4. Further, a power supply 43 is connected to the current perception threshold examination apparatus 4. A structure of the current perception threshold examination apparatus 4 will be described later.

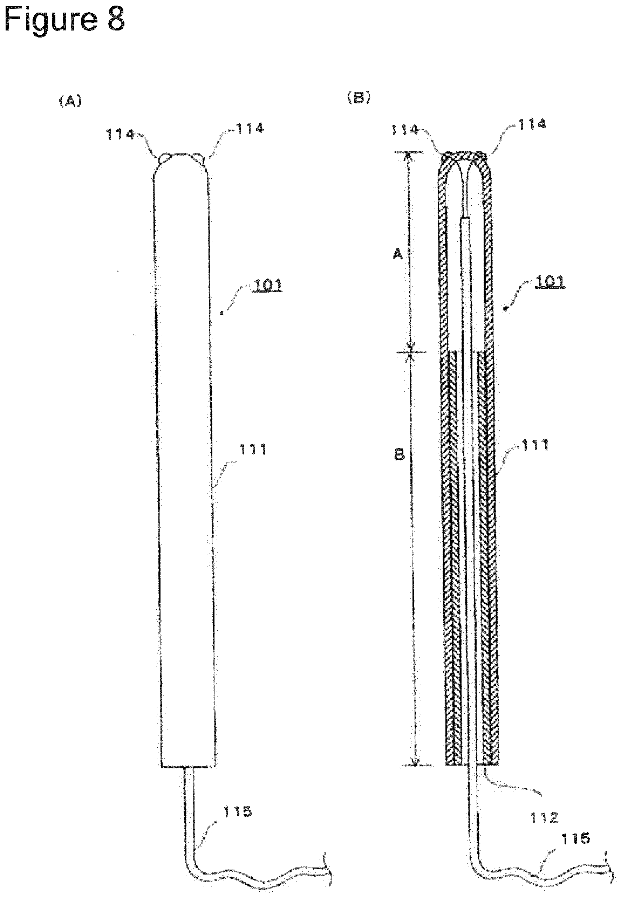

Further, another catheter for diagnosis of interstitial cystitis will be described on the basis of Figure 8 to Figure 13. Parts which are not specially described are the same as those in the catheter 1 for diagnosis of interstitial cystitis already described, and redundant explanation will be omitted.

As shown in Figures 8(A) and (B), a catheter 101 for diagnosis of interstitial cystitis mainly includes a catheter main body 111, a core member 112, a pair of electrodes 114, and lead wires 115. The catheter main body 111 is made of a soft flexible material (for example, a silicone rubber), and has a distal end section A that is inserted into a bladder and a main body section B adjacent to the distal end section A and at a proximal end side. The core member 112 is made of a rigid flexible member (for example, a polytetrafluoroethylene resin), and is inserted into a vicinity of a lower end of the distal end section A in the catheter main body 111.

The pair of electrodes 114 is provided on a distal end of the distal end section A of the catheter main body 111. The lead wires 115 are inserted into the core member, one ends are connected to the electrodes 114 and the other ends are drawn to outside from a proximal end portion of the catheter main body 111 to be connected to the current perception threshold examination apparatus 4 (refer to Figure 6).

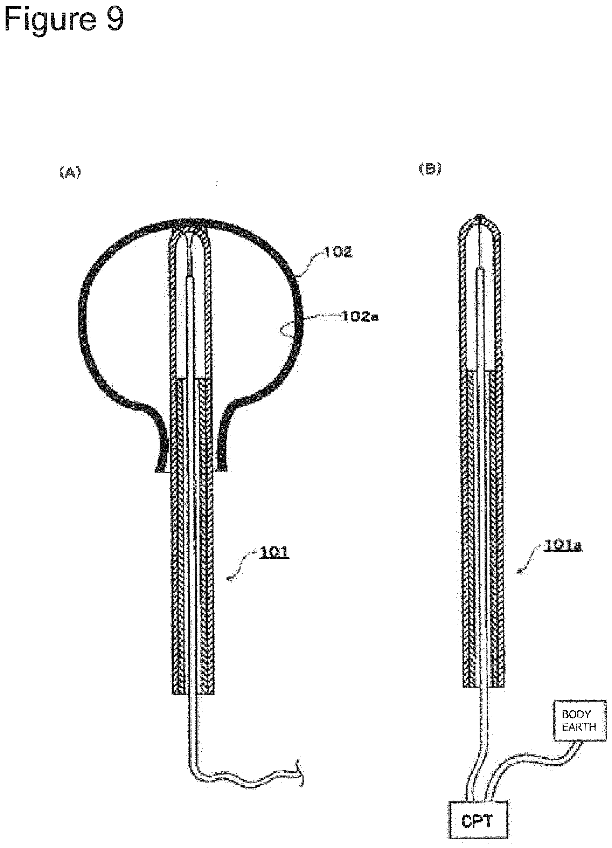

Figure 9(A) shows a state where the catheter 101 disclosed in Figure 8 is inserted into a bladder 102. As shown in this drawing, the catheter 101 is inserted to a position where a distal end of the catheter 101 contacts an inner wall 102a of the bladder 102. Subsequently, a current is caused to flow into the lead wires 115 of the catheter 101 from the current perception threshold examination apparatus 4. The current flows into the inner wall 102a of the bladder 2 from the electrodes 114 of the catheter 101.

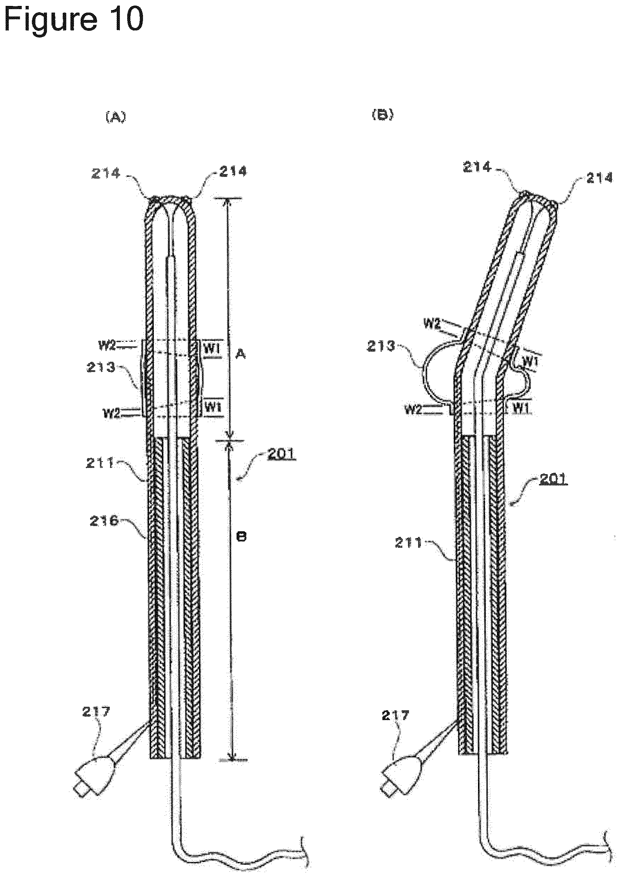

Further, Figure 9(B) is a catheter 101a which is made by modifying the catheter in Figure 9(A). In the catheter 101a, one electrode is provided at a distal end of the catheter main body, and another electrode is a body earth connected to the CPT. In actual use, the catheter 101a is inserted into the bladder of a patient who undergoes diagnosis, and the body earth is brought into contact with any part of the patient body. Thereby, a closed circuit is formed between the electrodes, and diagnosis of interstitial cystitis can be performed similarly to the catheter 101 in Figure 9(A). It is needless to say that the modification can be similarly applied to the catheters described in Figure 10 and the following drawings.

Next, on the basis of Figure 10 to Figure 13, still another catheter 201 for diagnosis of interstitial cystitis will be described. The catheter 201 differs from the first embodiment in a point that the catheter 201 has a predetermined balloon 213 on an outer circumferential surface of a catheter main body 211. That is, the cylindrical balloon 213 which is in such a manner as to cover the outer circumferential face of the catheter main body 211 is attached to a lower end of the distal end section A of the catheter main body 211. The balloon 213 is bonded onto the outer circumferential face of the catheter main body 211 by means of an adhesive in an upper end portion and a lower end portion respectively. Bonding widths thereof differ in accordance with locations in a circumferential direction. That is, as shown in Figure 10(A), the balloon 213 is bonded with a bonding width W1 at one side in a diameter direction (a right side in Figure 10(A)). The balloon 213 is bonded with a bonding width W2 at the other side in the diameter direction (a left side in Figure 10(A)). In other words, the bonding width continuously changes from W1 to W2 along a circumferential direction of the outer circumferential face of the catheter main body 211.

Further, at a proximal end side of the catheter main body 211, an injection portion 217 is provided. The injection portion 217 is for inflating the balloon 213 described above. Further, a fluid supply passage 216 that allows the injection portion 217 and an interior of the balloon 213 to communicate with each other is formed in the cylindrical wall which configures the catheter main body 211. From the injection portion 217, a fluid (for example, air, water or the like) is supplied to the balloon 213 through the fluid supply passage 216 to inflate the balloon 213.

When the fluid is supplied from the injection portion 217 in the catheter 201 as described above, the balloon 213 starts inflating. At this time, the bonding width of the balloon 213 is not uniform, and therefore, as shown in Figure 10(B), a degree of inflation differs depending on spots. Since in the example shown in the drawing, the right side has a larger bonding width, the balloon 213 is restrained in a wide range, and as a result, the balloon 213 has a smaller degree of inflation at the right side than at the left side. Here, the catheter main body 211 is formed from a soft flexible material, and therefore, can be deformed by an external force. Since the balloon 213 inflates non-uniformly with respect to the catheter main body 211 of the soft flexible material in this way, a non-uniform external force is applied to the catheter main body 211. Consequently, the catheter main body 211 is bent as shown in Figure 10(B) by a non-uniform external force like this.

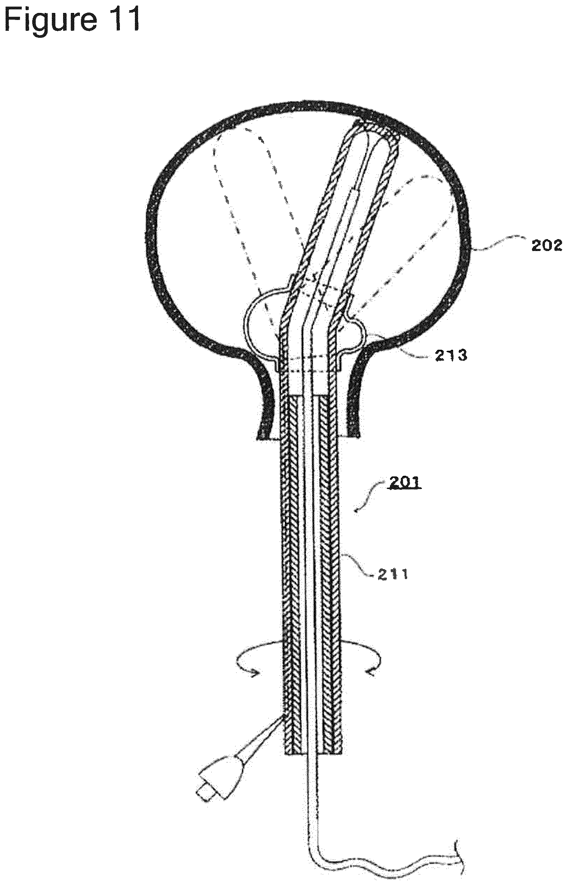

Figure 11 is a view showing a state where an inner wall of a bladder 202 is diagnosed by using the catheter 201 shown in Figure 10. As shown in the drawing, the catheter main body 211 is bent, and therefore, sites other than an uppermost portion of the inner wall of the bladder 202 can also be diagnosed. Further, by rotating the catheter main body 211 with an axis in a longitudinal direction as a center, it is possible to make diagnosis of a wide range. In particular, in accordance with an amount (for example, 1 cc to 4 cc) of the fluid which is injected into the balloon 213, the external force applied to the catheter main body 211 changes, and therefore, an angle at which the catheter main body 211 bends also changes (for example, 30° to 50°). Thereby, diagnosis of a desired inner wall of a bladder is enabled. Like this, in the state where the fluid is not injected, the catheter is not bent. Accordingly, it is easy to insert the catheter to the bladder.

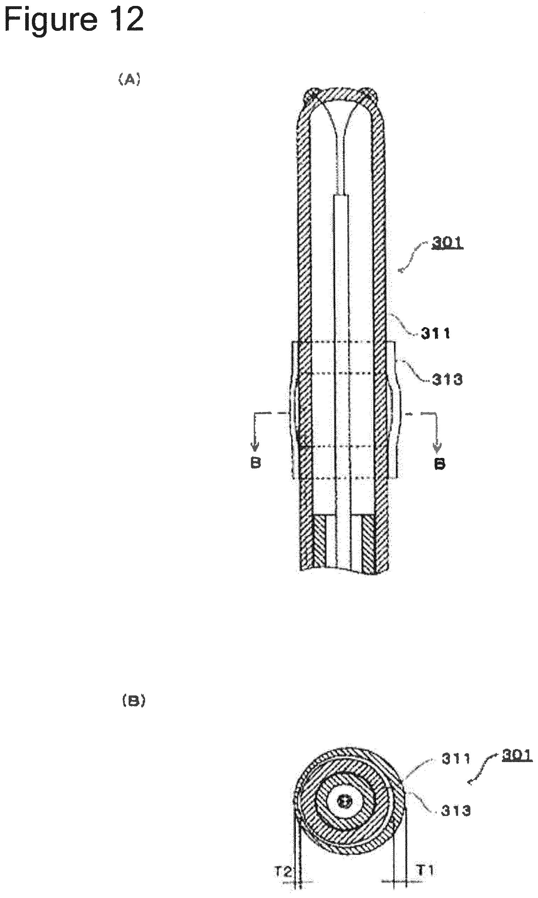

Figure 12 shows a modification example of the catheter for diagnosis of interstitial cystitis. In the modification example, a structure of a balloon 313 is different. That is, a thickness of the balloon 313 differs depending on locations. As shown in the drawing, a thickness T1 at a right side of the balloon 313 is larger than a thickness T2 at a left side. Consequently, when a fluid is injected into the balloon 313, the left side inflates more than the right side. As a result, an external force applied to the catheter main body 311 also becomes non-uniform, and the catheter main body 311 is bent. Here, a bending angle of the catheter main body 311 is desirably capable of being set in accordance with the injection amount of the fluid. More specifically, when the injection amount of the fluid to the balloon changes between 1 cc and 4 cc, for example, the bending angle preferably changes between approximately 30° and 50.

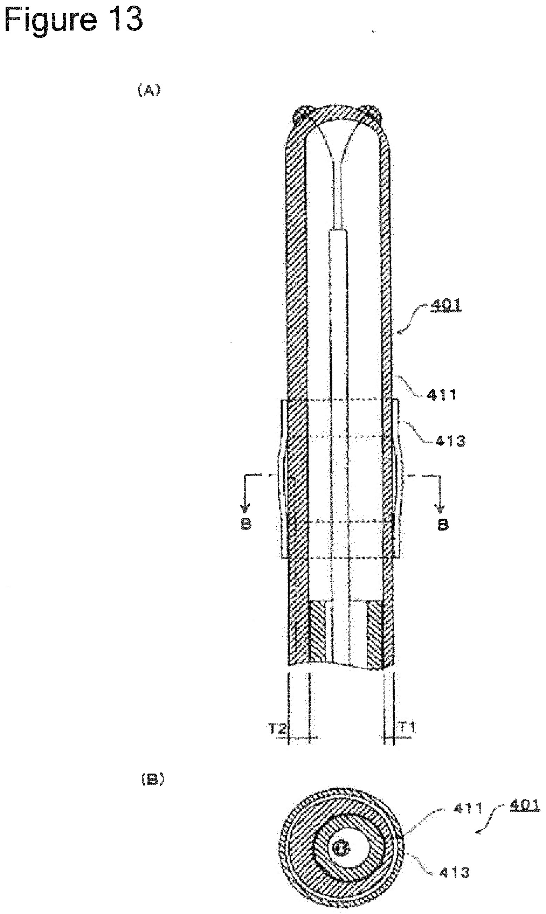

Figure 13 also shows another modification example of the catheter for diagnosis of interstitial cystitis. In the modification example, a structure of a catheter main body 411 is different. That is, a thickness of an outer circumferential wall of the catheter main body 411 differs depending on locations. As shown in Figure 13, the thickness T1 at a right side of the catheter main body 411 is thinner than the thickness T2 at a left side. Consequently, when a fluid is injected into a balloon 413, the right side deforms more greatly than the left side. As a result, even if an external force applied by the balloon 413 is uniform, the catheter main body 411 is bent.

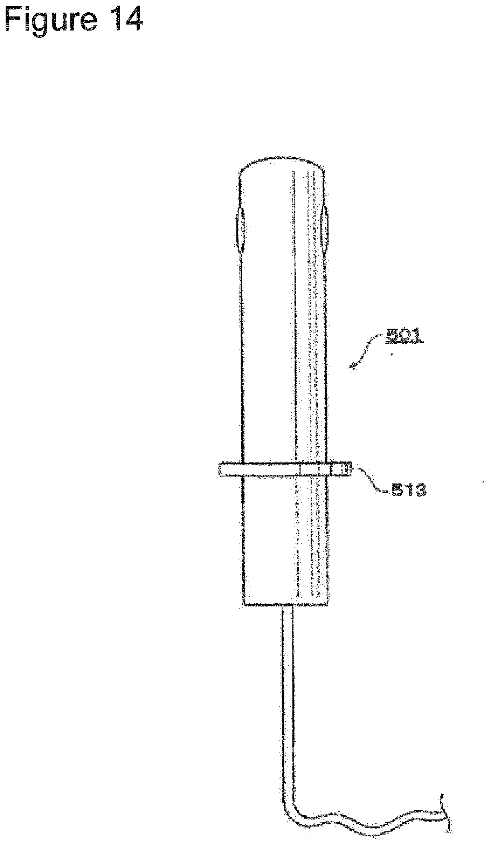

Figure 14 is a view showing a catheter in which electrodes are provided on side faces of a catheter main body. A catheter 501 is not for use in bladder diagnosis, but for use in vaginal diagnosis. Therefore, the catheter 501 is thicker as compared with the catheter for bladder diagnosis, and includes a ring-shaped flange 513 for restricting an insertion amount. By diagnosing a bladder with a catheter for bladder diagnosis, and performing diagnosis with the vaginal catheter, diagnosis of interstitial cystitis can be performed more precisely.

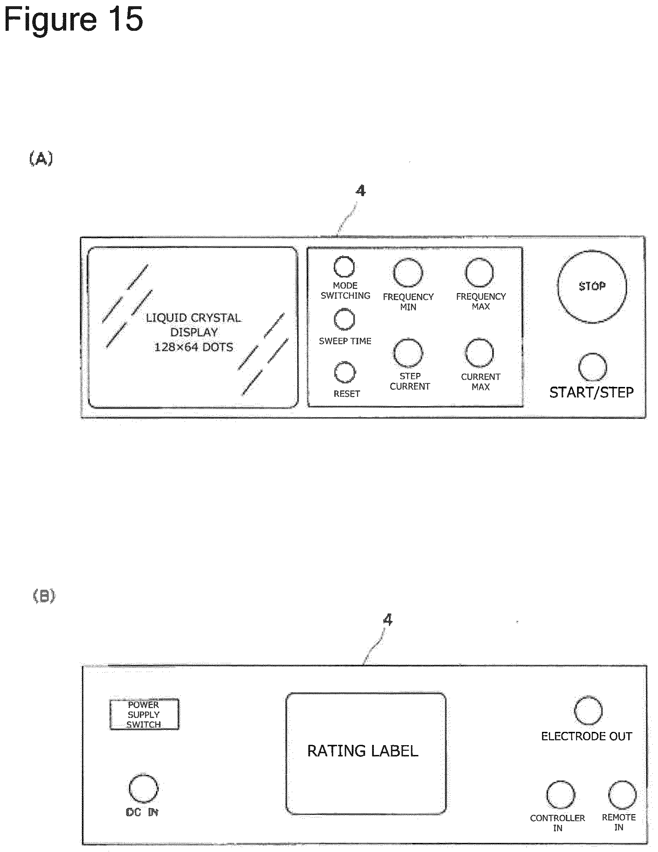

Next, on the basis of the drawings, the current perception threshold examination apparatus 4 according to one embodiment of the present invention will be described. Figure 15(A) shows a front view of the current perception threshold examination apparatus 4. As is understandable from the drawing, a liquid crystal display for information display is provided at a front face of the current perception threshold examination apparatus 4. The liquid crystal display displays a state of the current perception threshold examination apparatus 4, a diagnosis situation, and the like. More specifically, the liquid crystal display is configured by pixels of 128 by 64 dots. Although in the example shown in Figure 6, the current perception threshold examination apparatus 4 and a display unit 42 are made separate, this is only one example, and it is needless to say that the current perception threshold examination apparatus 4 and the display unit 42 may be configured to be integrated with each other, as shown in Figure 15.

Further, in the current perception threshold examination apparatus 4 is provided with respective buttons of "START/STEP", "STOP", "FREQUENCY MAX", "FREQUENCY MIN", "CURRENT MAX", "STEP CURRENT", "MODE SWITCHING", "SWEEP TIME", and "RESET".

The "START/STEP" button is pressed at a time of starting measurement, and by repeatedly pressing the button, start and interruption of each of diagnosis processes are enabled. For example, when the "START/STEP" button is pressed once, all the diagnosis processes automatically advance. When the button is pressed once more, the diagnosis process is intermitted at the point of the time. By pressing the button again, the diagnosis process is restarted from the interrupted process.

The "STOP" button is a button that is pressed at a time of completely stopping diagnosis. Even during diagnosis, all diagnosis processes which have been performed so far are all cleared by the button being pressed, and the next diagnosis is started from the initial diagnosis process.

The "FREQUENCY MAX" button is a button for making the frequency of the current which is supplied to the catheter maximum. In the current perception threshold examination apparatus 4 according to the present embodiment, 250 Hz is a maximum frequency although it is only an example.

The "FREQUENCY MIN" button is a button for making the frequency of the current which is supplied to the catheter minimum. In the current perception threshold examination apparatus 4 according to the present embodiment, a minimum frequency is 1 Hz although it is only an example.

The "CURRENT MAX" button is a button for making the current which is supplied to the catheter maximum. In the current perception threshold examination apparatus 4 according to the present embodiment, a maximum current value is 5 mA although it is only an example.

The "STEP CURRENT" button is a button for changing the value of the current which is supplied to the catheter in a step manner. In the current perception threshold examination apparatus 4 according to the present embodiment, a current value resolution at each step differs depending on preliminary examination or regular diagnosis. In the preliminary examination, the value of the current changes every 200 µA, and in the regular diagnosis, the value of the current changes every 10 µA.

The "MODE SWITCHING" button is a button for switching whether to manually change or automatically change the frequency of the current which is supplied to the catheter. In the case of a manual mode, the frequency changes every 1 Hz, and in the case of an automatic mode, the frequency changes continuously.

The "SWEEP TIME" button is for setting a diagnosis time period at a time of performing diagnosis by automatically changing the frequency. For example, when diagnosis is performed precisely, a sweep time needs to be set to be long, whereas when rough diagnosis can be sufficient, the sweep time may be set to be short. It is possible to set a unit sweep time by pressing the button once, and it is possible to set the sweep time to be long by pressing the button a plurality of times.

The "RESET" button is a button for returning all conditions set to the current perception threshold examination apparatus 4 in diagnosis to initial states. For example, control of making the current value and the frequency minimum, switching change of the frequency to the manual mode and the like is performed. Further, stored diagnosis data is all cleared. However, only the set items that differ according to diagnosis are returned to the initial states, and more basic set items are not initialized.

Further, as shown in Figure 15(B), a power supply switch, a DC IN terminal, an electrode OUT terminal, a controller IN terminal and a remote IN terminal are provided on a back face of the current perception threshold examination apparatus 4. The power supply switch is a switch for actuating the current perception threshold examination apparatus 4. The DC IN terminal is a terminal to which a connection terminal of a power supply (a power supply adapter) 43 is connected. The power supply 43 of the present embodiment has an output of DC 12 V/12 W with respect to an input of AC 90 to 264 V and 47 to 63 Hz. The electrode OUT terminal is a terminal to which lead wires for electrodes of the catheter are connected. The controller IN terminal is a terminal for connecting a controller operated by a diagnostician. Further, the remote IN terminal is a terminal for connecting a remote switch that is operated by an examinee when the examinee feels a stimulus.

An example of specifications of the current perception threshold examination apparatus 4 according to the present embodiment is as follows. First, a rated voltage for measurement is 12 V, and an output frequency is variable between 1 and 250 Hz. As operation modes, a manual diagnosis mode by steps at intervals of 1 Hz, and a frequency automatically variable diagnosis mode are available. The output current value is switchable in two stages of 2.5 mA and 5 mA, and a limiter is operated at 5.5 mA. As the measurement resolution, 10 µA and 200 µA are selectable.

The current perception threshold examination apparatus 4 has an information storage unit, which can store information of 100 events, and stores information of a name of a diagnostician, a name of an examinee, a date and time, a frequency value, a current value, a voltage value and the like, for one event.

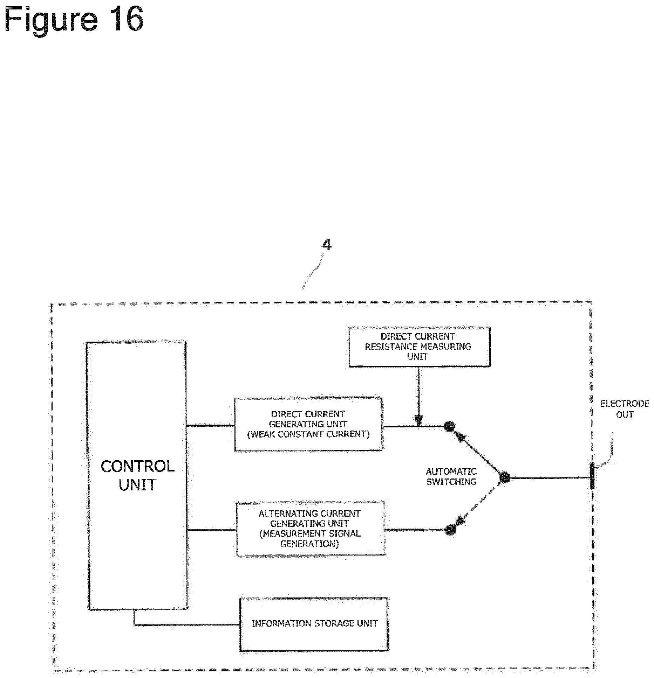

Further, as shown in Figure 16, the current perception threshold examination apparatus 4 is provided with a control unit that controls supply of a current to the electrodes of the catheter, a direct current generating unit that generates a direct current in response to an instruction from the control unit, and an alternating current generating unit that similarly generates an alternating current. The direct current generating unit supplies a weak direct current to the electrodes of the catheter in response to the instruction of the control unit, and measures an electric resistance value of a diagnosis site of an examinee. The electric resistance value serves as reference data for determining a proper voltage value at a time of applying an alternating current to the diagnosis site, as described later. Further, the alternating current generating unit generates an alternating current for use in actual diagnosis of the diagnosis site of the examinee, in response to an instruction from the control unit.

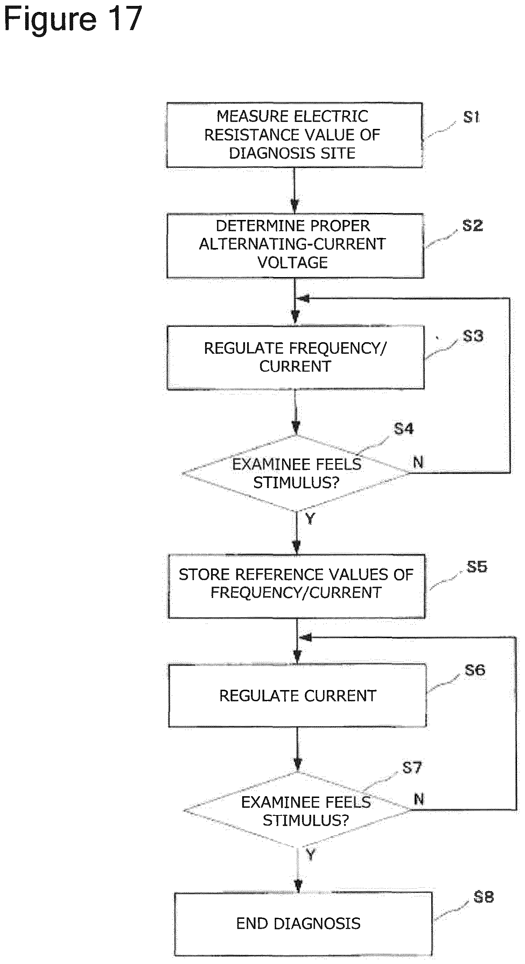

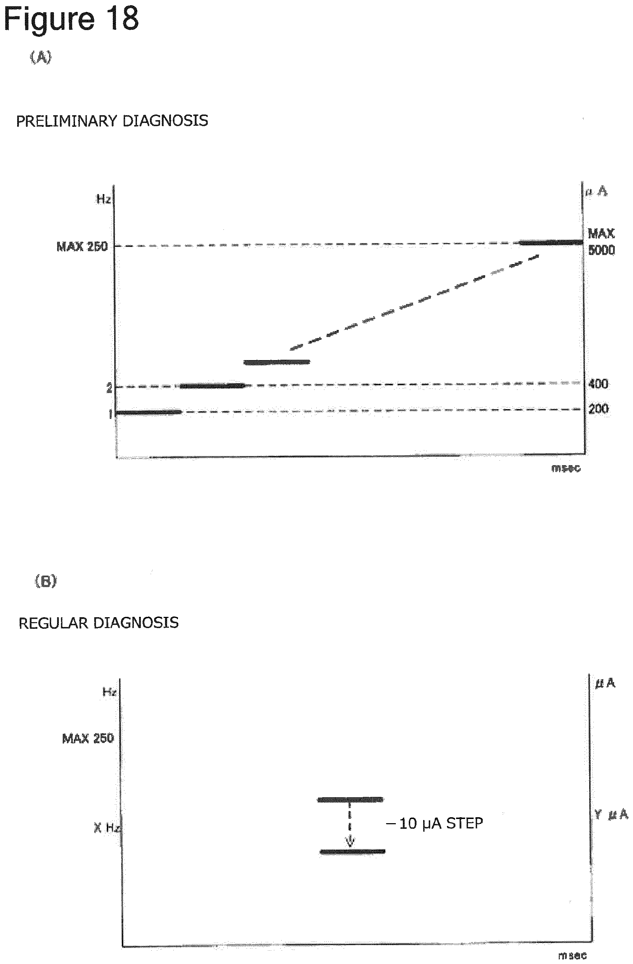

Next, on the basis of Figure 17 and Figure 18, actual measurement (diagnosis) will be described. First, the control unit issues an instruction to the direct current generating unit so as to generate a direct current. In response to this, the direct current generating unit generates a weak direct current. The weak direct current is supplied to the electrodes through the catheter, and by the direct current resistance measuring unit which is attached to the direct current generating unit, the electric resistance value between the electrodes through the diagnosis site is measured (step S1). When the electric resistance value is measured, a proper alternating-current voltage value to be applied to the diagnosis site can be determined (step S2).

Next, the flow goes to a process of preliminary examination. In the preliminary examination, the direct current generating unit is switched to the alternating current generating unit. At this time, the alternating current generating unit applies the alternating-current voltage which is determined on the basis of the aforementioned electric resistance value to the electrodes, in response to the instruction from the control unit. Meanwhile, a current is set as follows. That is, as shown in Figure 18(A), a frequency of the alternating current is set at 1 Hz, and an effective value of the current is set to be 200 µA (step S3).

When the examinee does not feel pain (step S4), the frequency is set at 2 Hz, and the current value is set at 400 µA (step S3). In other words, every time the frequency is increased by 1 Hz, the current which is supplied is increased by 200 µA. A maximum is 5000 µA (5 mA) with 250 Hz. When the examinee feels pain before the maximum value is obtained, reference values of the frequency and the current value at this time are stored in the information storage unit (step S5).

Next, the flow goes to regular diagnosis. In the regular diagnosis, the frequency stored in the information storage unit is read, and after the frequency is fixed to the value, the current value is increased and decreased by 10 µA with the stored current value as a center (refer to step S6 in Figure 17, and Figure 18(B)), to narrow down a current value at which the examinee feels pain (step S7). Subsequently, a current value in a boundary at which the examinee feels and doesn't feel pain is determined, and diagnosis of interstitial cystitis is ended (step S8).

### INDUSTRIAL APPLICABILITY

The present invention can be used in a current perception threshold examination apparatus which is used in a catheter for diagnosis of interstitial cystitis.

### REFERENCE SIGNS LIST

- 1: Catheter for diagnosis of interstitial cystitis
- 2: Bladder
- 4: Current perception threshold examination apparatus
- 11: Catheter main body
- 13: Balloon
- 14: Electrode
- 16: Fluid supply passage
- 17: Injection portion
- 101: Catheter for diagnosis of interstitial cystitis
- 102: Bladder
- 111: Catheter main body
- 114: Electrode
- 115: Lead wire
- A: Distal end section
- B: Main body section

## Claims

1. A current perception threshold examination apparatus (4) that controls supply of a current from a power supply (43) to electrodes (14, 114) of a catheter (1, 101, 201, 301, 401, 501) for diagnosis of interstitial cystitis,
the current perception threshold examination apparatus (4) comprising: a direct current generating unit for generating a direct current on the basis of an instruction of a control unit; a direct current resistance measuring unit that measures an electric resistance value of a diagnosis site on the basis of the direct current which is supplied to the electrodes; and an alternating current generating unit for generating an alternating current to be supplied for diagnosis, on the basis of the electric resistance value which is measured.

2. The current perception threshold examination apparatus according to claim 1,
wherein the control unit determines an alternating-current voltage value suitable for diagnosis on the basis of the electric resistance value, and the alternating current generating unit applies the determined voltage to the electrodes (14, 114) in response to an instruction of the control unit.

3. The current perception threshold examination apparatus according to claim 2,
wherein the control unit has a frequency and current value regulating function of regulating a frequency and a current value.

4. The current perception threshold examination apparatus according to claim 3,
wherein the diagnosis of interstitial cystitis is divided into preliminary examination and regular diagnosis, and the control unit further has a resolution switching function of switching a resolution for a current value in the preliminary examination and the regular diagnosis.

5. The current perception threshold examination apparatus according to claim 4,
wherein a resolution in the regular diagnosis is higher than a resolution in the preliminary examination.

## Patentansprüche

1. Vorrichtung zur Untersuchung einer Stromerfassungsschwelle (4), die die Lieferung eines Stroms von einer Stromversorgung (43) an die Elektroden (14, 114) eines Katheters (1, 101, 201, 301, 401, 501) zur Diagnose von interstitieller Zystitis steuert,
wobei die Vorrichtung zur Untersuchung einer Stromerfassungsschwelle (4) umfasst: eine Gleichstromerzeugungseinheit zum Erzeugen eines Gleichstroms auf der Basis einer Anweisung einer Steuereinheit; eine Gleichstromwiderstandsmesseinheit, die einen elektrischen Widerstandswert einer Diagnosestelle auf der Basis des Gleichstroms berechnet, der an die Elektroden geliefert wird; und eine Wechselstromerzeugungseinheit zum Erzeugen eines zur Diagnose zu liefernden Wechselstroms auf der Basis des elektrischen Widerstandswerts, der gemessen wird.

2. Vorrichtung zur Untersuchung einer Stromerfassungsschwelle nach Anspruch 1,
wobei die Steuereinheit einen zur Diagnose geeigneten Wechselstromspannungswert auf der Basis des elektrischen Widerstandswerts bestimmt, und die Wechselstromerzeugungseinheit die bestimmte Spannung in Reaktion auf eine Anweisung der Steuereinheit an die Elektroden (14, 114) anlegt.

3. Vorrichtung zur Untersuchung einer Stromerfassungsschwelle nach Anspruch 2,
wobei die Steuereinheit eine Frequenz- und Stromwertregelfunktion zum Regeln einer Frequenz und eines Stromwerts aufweist.

4. Vorrichtung zur Untersuchung einer Stromerfassungsschwelle nach Anspruch 3,
wobei die Diagnose von interstitieller Zystitis in eine Voruntersuchung und eine reguläre Diagnose unterteilt ist, und die Steuereinheit ferner eine Auflösungsschaltfunktion zum Schalten einer Auflösung für einen Stromwert in der Voruntersuchung und der regulären Diagnose aufweist.

5. Vorrichtung zur Untersuchung einer Stromerfassungsschwelle nach Anspruch 4,
wobei eine Auflösung in der regulären Diagnose höher ist als eine Auflösung in der Voruntersuchung.

## Revendications

1. Appareil pour l'examen du seuil de perception du courant (4) commandant l'alimentation d'un courant à partir d'une source d'énergie (43) à des électrodes (14, 114) d'un cathéter (1, 101, 201, 301, 401, 501) pour le diagnostic de la cystite interstitielle,
l'appareil pour l'examen du seuil de perception du courant (4) comprenant : une unité de génération de courant continu pour générer un courant continu sur la base d'une instruction d'une unité de commande ; une unité de mesure de résistance au courant continu qui mesure une valeur de résistance électrique d'un site de diagnostic sur la base du courant continu qui est fourni aux électrodes ; et une unité de génération de courant alternatif pour générer un courant alternatif devant être fourni pour le diagnostic, sur la base de la valeur de résistance électrique qui est mesurée.

2. Appareil pour l'examen du seuil de perception du courant selon la revendication 1,
l'unité de commande déterminant une valeur de tension de courant alternatif appropriée pour le diagnostic sur la base de la valeur de résistance électrique, et l'unité de génération de courant alternatif appliquant la tension déterminée aux électrodes (14, 114) en réponse à une instruction de l'unité de commande.

3. Appareil pour l'examen du seuil de perception du courant selon la revendication 2,
l'unité de commande ayant une fonction de régulation de la fréquence et de la valeur du courant pour réguler une fréquence et une valeur de courant.

4. Appareil pour l'examen du seuil de perception du courant selon la revendication 3,
le diagnostic de la cystite interstitielle étant divisé en un examen préliminaire et un diagnostic régulier, et l'unité de commande ayant en outre une fonction de commutation de résolution pour commuter une résolution pour une valeur de courant dans l'examen préliminaire et le diagnostic régulier.

5. Appareil pour l'examen du seuil de perception du courant selon la revendication 4,
une résolution dans le diagnostic régulier étant supérieure à une résolution dans l'examen préliminaire.
